# DEMANDE DE BREVET EUROPEEN

(11) **EP 0 826 345 A1**
(43) Date de publication de la demande: **04.03.1998**
(21) Numéro de dépôt: 97401977.0
(22) Date de dépôt: 22.08.1997
(51) Int. Cl.: A61C 13/00, A61C 19/04, A61C 9/00

(54) **Dispositifs de marquage et de repérage d'une zone à traiter de la mâchoire d'un être vivant**

(30) Priorité: 26.08.1996 FR 9610446
(71) Demandeur: Derycke, Raymond-René, 75116 Paris (FR)
(72) Inventeur: Derycke, Raymond-René, 75116 Paris (FR)
(74) Mandataire: Moncheny, Michel

(57) **Abrégé**

Le dispositif de marquage (41) comporte un ensemble de marques (40) pouvant apparaître sur une image produite par un dispositif d'imagerie médicale (10). Des moyens de fixation amovible dudit ensemble sur une mâchoire (18) d'un être vivant dans une position déterminée, et des moyens de positionnement ultérieur dudit dispositif dans ladite position déterminée après un retrait de celui-ci sont en outre prévus.

Le dispositif de repérage (34) comporte d'une part un ensemble de points de référence, comportant des éléments (34A, 34B, 34C) détectables par un dispositif (12) de positionnement d'un objet dans l'espace, et d'autre part des moyens de fixation mécanique de cet ensemble de points de référence sur une mâchoire (18) d'un être vivant.

## Description

La présente invention concerne un dispositif de marquage et un dispositif de repérage destinés à être installés sur la mâchoire d'un être vivant en vue de l'utilisation d'une installation de contrôle visuel de la position d'un outil travaillant dans cette mâchoire.

Lors de la réalisation d'opérations chirurgicales, il est fréquent que le chirurgien doive suivre par un procédé de stéréotaxie, le mouvement de l'outil qu'il manie sur un écran de visualisation.

A cet effet, il utilise une installation d'imagerie médicale pour l'obtention d'images numérisées de l'organe devant être traité par l'outil. Un dispositif de positionnement de l'outil dans l'espace est en outre mis en oeuvre. Il permet de déterminer en continu la position de l'outil par rapport à l'organe traité. Ce dispositif de positionnement de l'outil est relié à une unité centrale de traitement d'informations comportant des moyens d'affichage d'un représentant graphique de l'outil sur une image numérique de l'organe. Les images numérisées qui sont produites par l'installation d'imagerie médicale sont préalablement chargées et mémorisées dans ladite unité centrale de traitement d'informations en vue de leur affichage.

Le dispositif de positionnement de l'outil nécessite la connaissance de points de référence fixes.

Ces points de référence sont généralement pris sur une structure fixe disposée à proximité du patient. Le patient, et en particulier l'organe à traiter, doivent être immobilisés par rapport à cette structure fixe.

En effet, tout déplacement relatif entre l'organe à traiter et les points de référence conduirait à une représentation erronée de la position de l'outil par rapport à l'organe.

Ainsi, les installations de l'état de la technique sont peu fiables et ne sont pas utilisables pour des organes ne pouvant pas être immobilisés par rapport à une structure fixe.

En particulier, dans le cas de la chirurgie dentaire, où il est impossible d'immobiliser une mâchoire du patient lors de l'opération, une telle installation ne peut être mise en oeuvre.

De plus, il est difficile dans les installations actuelles de repérer avec précision, par rapport aux points de référence, la zone de l'organe devant être traitée, et en particulier de déterminer la position exacte des images de la zone à traiter par rapport à cet ensemble de points de référence.

Une solution a été proposée dans le cas de la chirurgie crânienne. Elle consiste à placer à demeure un ensemble de marques pouvant apparaître sur des images produites par le dispositif d'imagerie médicale. Ces marques permettent le positionnement des images numériques prises préalablement par rapport à l'ensemble de points de référence.

Avec cette technique, les marques restent fixées sur le crâne entre le moment où les images numériques sont prises et le moment où l'intervention chirurgicale est pratiquée. Un délai de plusieurs jours peut s'écouler entre ces deux opérations. Aussi, de telles marques ne sont pas adaptées à la chirurgie dentaire puisqu'il est impossible de laisser celles-ci implantées plusieurs jours dans la bouche du patient avant de pratiquer l'intervention chirurgicale.

Les dispositifs de marquage et de repérage connus ne sont donc pas adaptés à la chirurgie dentaire avec l'assistance de moyens de visualisation de l'outil.

L'invention a pour but de proposer des dispositifs de marquage et de repérage permettant de visualiser avec précision à partir d'une installation de contrôle visuel un outil dans la mâchoire d'un être vivant lors du traitement chirurgical, qui ne présentent pas les inconvénients mentionnés ci-dessus.

A cet effet, l'invention a pour objet un dispositif de marquage caractérisé en ce qu'il comporte un ensemble de marques pouvant apparaître sur une image produite par un dispositif d'imagerie médicale, des moyens de support amovible dudit ensemble de marques sur une mâchoire d'un être vivant dans une position déterminée, et des moyens de positionnement ultérieur dudit ensemble de marques dans ladite position déterminée après un retrait du dispositif.

Suivant des modes particuliers de réalisation, le dispositif peut comporter une ou plusieurs caractéristiques suivantes :
- lesdits moyens de positionnement ultérieur comportent une empreinte permanente d'une partie de la mâchoire prise lors de la première installation du dispositif, ladite empreinte pouvant être remise en place sur ladite partie de la mâchoire lors de l'installation ultérieure du dispositif afin d'assurer le positionnement ultérieur de l'ensemble de marques dans ladite position déterminée,
- lesdits moyens de support comportent une gouttière sur laquelle sont disposées lesdites marques, et lesdits moyens de positionnement ultérieur comportent un matériau plastiquement déformable disposé dans ladite gouttière pour la prise de ladite empreinte,
- il comporte des moyens de fixation amovible du dispositif,
- lesdits moyens de fixation comportent des organes de serrage destinés à enserrer ladite mâchoire,
- lesdits organes de serrage comportent des vis montées dans des trous taraudés traversant des parois latérales de la gouttière, l'extrémité libre des vis étant destinée à coopérer avec ladite mâchoire, et
- il comporte en outre un ensemble de points de référence, comportant des éléments détectables par un dispositif de positionnement d'un objet dans l'espace, lequel ensemble de points de référence est porté par des moyens de support dudit ensemble de marques sur la mâchoire.

L'invention a également pour objet un dispositif de repérage, caractérisé en ce qu'il comporte d'une part un ensemble de points de référence, comportant des éléments détectables par un dispositif de positionnement d'un objet dans l'espace, et d'autre part des moyens de fixation mécanique de cet ensemble de points de référence sur une mâchoire d'un être vivant.

Ce dispositif peut comporter l'une ou plusieurs des caractéristiques suivantes :
- il comporte un prolongement rigide reliant lesdits éléments ponctuels auxdits moyens de fixation, lequel prolongement rigide est destiné à traverser la bouche de l'être vivant afin de déporter lesdits éléments ponctuels à l'extérieur du corps dudit être vivant,
- lesdits moyens de fixation comportent d'une part, une gouttière destinée à chevaucher un secteur de la mâchoire dudit être vivant, et d'autre part, des organes de serrage destinés à enserrer ladite mâchoire pour retenir la gouttière sur celle-ci,
- lesdits organes de serrage comportent des vis montées dans des trous taraudés traversant des parois latérales de la gouttière, l'extrémité libre des vis étant destinée à coopérer avec ladite mâchoire,
- lesdits moyens de fixation comportent au moins un support fileté destiné à être vissé dans une douille implantée dans la mâchoire dudit être vivant, et
- il comporte un ensemble de marques pouvant apparaître sur une image produite par un dispositif d'imagerie médicale, lesdits moyens de fixation mécanique comportant des moyens de support dudit ensemble de marques sur ladite mâchoire dans une position déterminée et il comporte en outre des moyens de positionnement ultérieur dudit ensemble de marques dans ladite position déterminée après un retrait du dispositif.

L'invention sera mieux comprise à la lecture de la description qui va suivre, donnée uniquement à titre d'exemple et faite en se référant aux dessins sur lesquels :
- la Fig.1 est une schéma d'une installation de contrôle visuel de la position d'un outil dans une mâchoire d'un être vivant équipé de dispositif de marquage et de repérage selon l'invention ;
- la Fig.2 est une vue en coupe transversale du dispositif de marquage selon l'invention ;
- la Fig.3 est une vue en perspective du dispositif de marquage de la Fig.2 après une première mise en place sur une mâchoire ;
- la Fig.4 est une vue en perspective d'un dispositif de repérage selon l'invention ;
- les Fig.5 à 7 sont des vues en coupe transversale de trois variantes de réalisation d'un dispositif de repérage monté sur une mâchoire humaine; et
- la Fig.8 est une vue en perspective d'un autre mode de réalisation du dispositif de repérage selon l'invention.

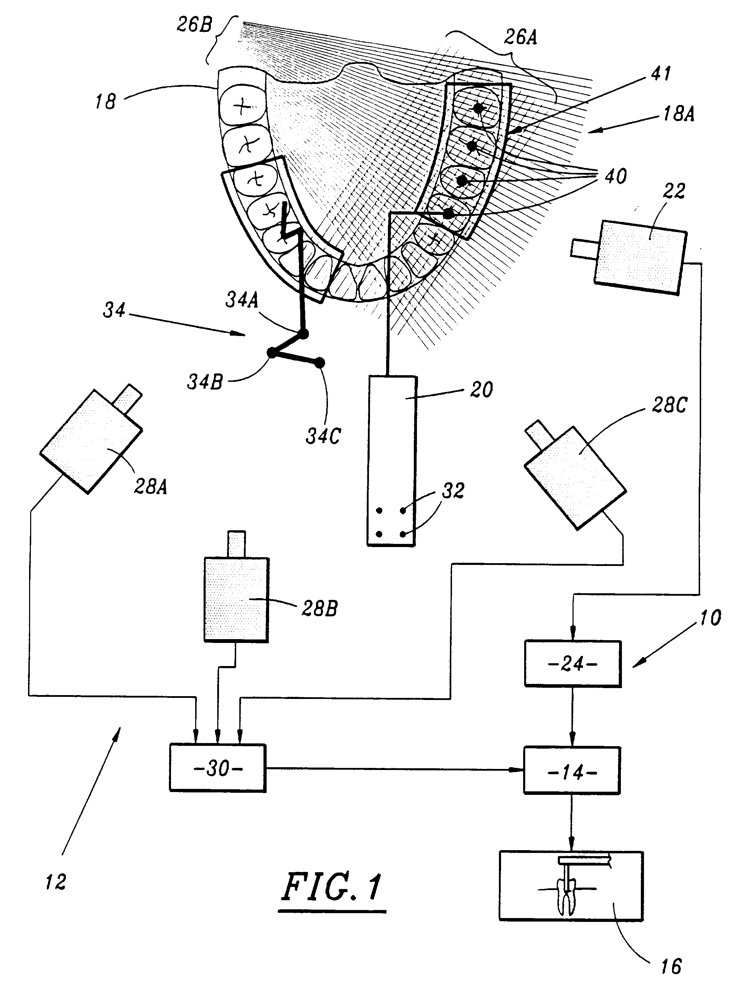

L'installation de contrôle visuel représentée sur la figure 1, comporte essentiellement un dispositif d'imagerie médicale 10, un dispositif 12 de positionnement d'un objet dans l'espace, une unité centrale de traitement d'informations 14 à laquelle ils sont tous deux reliés, et des moyens d'affichage 16.

Sur cette figure, sont en outre représentés en vue de dessus une mâchoire par exemple inférieure 18 d'un être humain, et un outil 20 de traitement chirurgical de cette mâchoire. Cet outil 20 est par exemple une fraiseuse.

L'installation sera décrite dans la suite lors de son utilisation dans le cadre de la visualisation d'un outil de chirurgie dentaire. Toutefois, celle-ci pourrait être mise en oeuvre pour la visualisation d'un outil dans tout autre organe que la mâchoire.

Le dispositif d'imagerie médicale 10 comporte des moyens de prise d'images numériques 22, reliés à des moyens 24 de mémorisation des images numériques. Ces moyens de mémorisation d'images numériques sont reliés à l'unité centrale 14.

Les moyens 22 de prise d'images numériques sont adaptés pour effectuer un balayage transversal à pas constant de la mâchoire 18 dans une zone 18A devant être traitée, et ainsi, engendrer des faisceaux d'images 26A, 26B s'étendant dans des directions différentes.

Comme représenté sur la figure 1, les images successives du faisceau 26A s'étendent suivant des plans parallèles alors que les images du faisceau 26B sont prises suivant des plans rayonnants.

Le pas séparant chacun des plans successifs d'un même faisceau est par exemple de 0,5 mm.

De telles images peuvent être prises par exemple par une technique de type sonique ou électromagnétique (tomodensitométrie ou RMN) ou tout autre technique appropriée.

Le dispositif 12 de positionnement d'un objet dans l'espace comporte trois caméras infrarouges 28A, 28B, 28C, disposées extérieurement autour de la bouche du patient. Ces caméras sont reliées à une unité de traitement d'images 30 adaptée pour déterminer la position et l'orientation relative d'un objet par rapport à un repère de référence.

A cet effet, l'objet dont on souhaite repérer la position et l'orientation comporte à sa surface exposée plusieurs éléments ponctuels de positionnement, notamment quatre disposés en carré. Ces éléments de positionnement sont par exemple formés par des diodes émettrices infrarouges dont le rayonnement est susceptible d'être détecté par les caméras 28A à 28C. Dans l'exemple décrit, quatre diodes émettrices portant la référence 32 sont prévues sur le corps de l'outil 20.

Afin de permettre de positionner l'outil, un ensemble de points de référence est porté par un dispositif de repérage selon l'invention portant la référence 34. Ce dispositif 34 est fixé mécaniquement sur la mâchoire 18. Il sera décrit plus en détail dans la suite de la description.

Le procédé permettant le contrôle visuel de la position d'une tête d'outil, par exemple une fraise, travaillant dans la mâchoire 18 va maintenant être décrit.

Dans l'exemple décrit, l'outil est utilisé pour assurer le perçage de trous dans la mâchoire, en vue notamment de l'implantation de douilles de fixation de prothèses dentaires.

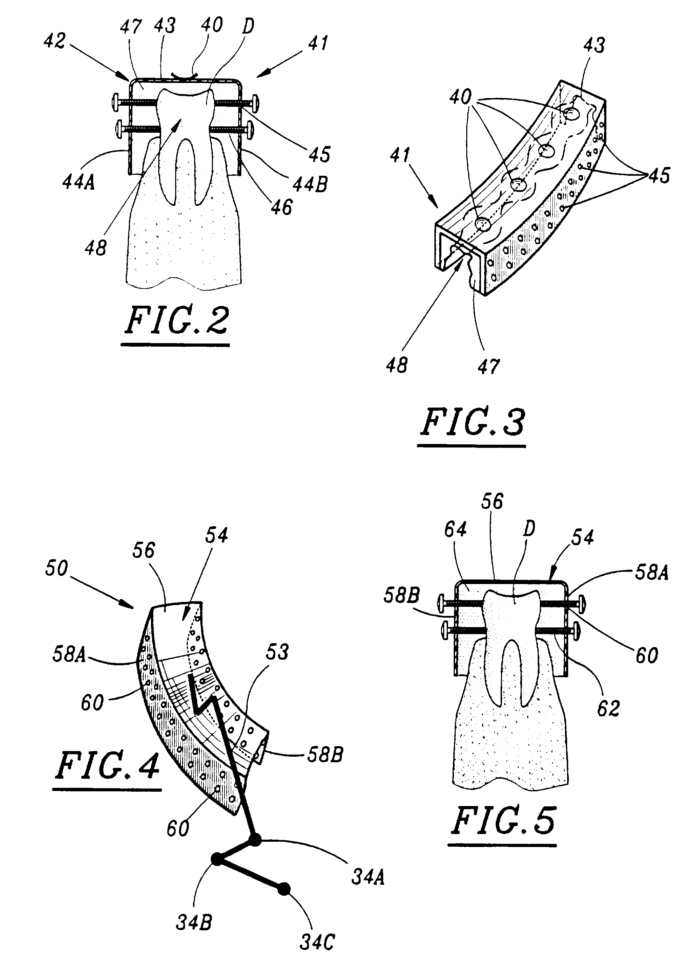

Le chirurgien place dans un premier temps un ensemble de marques 40 non alignées sur la surface supérieure de la mâchoire aux emplacements auxquels doivent déboucher des perçages. Ces marques sont portées par un dispositif de marquage 41 selon l'invention représenté sur les figures 2 et 3.

Comme représenté sur la figure 2, le dispositif de marquage comporte une gouttière courbe 42 de section en U, délimitée par une surface supérieure 43 sur laquelle sont collées les marques 40 et deux parois latérales 44A, 44B. Cette gouttière constitue un moyen de support des marques 40 sur la mâchoire. Tout autre moyen de support amovible adapté peut être utilisé.

Les parois latérales comportent un ensemble de trous taraudés 45 dans lesquels sont montées des vis 46. Les extrémités des vis 46 reçues à l'intérieur de la gouttière 42 prennent appui sur les parois latérales des dents D. Elles assurent ainsi l'immobilisation du dispositif de marquage 41 sur la mâchoire en enserrant celle-ci.

Les marques 40 sont formées par des pastilles légèrement bombées ayant une forme de coupelle. Les pastilles sont réalisées dans un matériau tel, que le dispositif d'imagerie médicale 10 utilisé détecte celles-ci et que les marques apparaissent sur les images numériques prises suivant des plans les contenant.

Tel que représenté sur les figures, quatre marques sont disposées sur la surface supérieure aux endroits auxquels le chirurgien devra procéder au perçage.

Par ailleurs, une pâte 47, formée d'un matériau plastiquement déformable, est disposée à l'intérieur de la gouttière 42. Cette pâte est destinée à la prise d'une empreinte 48 de la mâchoire, et en particulier des dents dans la zone devant être traitée par l'outil. Cette pâte est telle qu'elle se solidifie d'elle-même après la prise de l'empreinte.

Afin de procéder à la mise en place du dispositif de marquage 41, la gouttière 40 dans laquelle a été préalablement disposée la pâte 47 est appliquée fortement sur la mâchoire de sorte que les dents forment dans la pâte l'empreinte 48. Les vis 46 sont ensuite serrées contre les dents pour immobiliser le dispositif de marquage 41 sur la mâchoire.

Il est à noter que les moyens de fixation constitués par les vis 46 sont facultatifs, les marques pouvant être supportées uniquement par l'intermédiaire de la gouttière 42 et de la pâte 47.

La phase suivante consiste en la prise d'images numériques à partir du dispositif 10 d'imagerie médicale.

Alors que la mâchoire du patient est momentanément immobilisée, plusieurs faisceaux d'images numériques 26A, 26B sont réalisés. Le pas séparant les images étant suffisamment faible, les marques 40 apparaissent sur certaines des images numérisées. Les images ainsi numérisées sont ensuite stockées dans les moyens de mémorisation 24.

Après que les images ont été prises, le dispositif de marquage 41 est retiré de la mâchoire. Pour ce faire, les vis 46 sont desserrées et la gouttière 42 est extraite avec précaution de sorte que l'empreinte 48 de la mâchoire reste formée dans la pâte 47. Après retrait du dispositif de marquage, la pâte 47 durcit de sorte que l'empreinte 48 devient permanente.

Comme représenté sur la figure 3, le dispositif de marquage 41 comporte, après retrait et démontage des vis 46, l'ensemble des marques 40 fixées sur la surface supérieure 43 et l'empreinte 48 formée dans le matériau plastique 47.

Le patient reprend une vie normale jusqu'au jour de l'opération.

Avant l'opération, le dispositif de marquage 41 est remis en place dans la zone 18A devant être traitée. L'empreinte 48 permet le positionnement du dispositif de marquage 41 dans la position exacte dans laquelle il avait été mis en place la première fois lors de la réalisation des images de la zone à traiter. Les vis 46 sont à nouveau serrées sur les dents de sorte que le dispositif de marquage 41 est fixé à la mâchoire.

Le dispositif de repérage 34 portant un ensemble de points de référence 34A, 34B, 34C non alignés, fixes les uns par rapport aux autres, est ensuite mis en place sur la mâchoire 18. Ces trois points de référence 34A, 34B, 34C sont formés par exemple par des diodes émettrices infrarouges susceptibles d'être détectées par les caméras infrarouges 28A à 28C. Les points de référence 34A à 34C et les éléments de positionnement 32 sont de même nature.

Sur la figure 4 est représenté un exemple d'un dispositif de repérage adapté pour la chirurgie dentaire. Il comporte d'une part des moyens 50 de fixation sur la mâchoire du patient et d'autre part les points de référence 34A à 34C formés par des diodes émettrices. Ces dernières sont disposées non alignées à l'extrémité d'un prolongement rigide formé d'une tige 53 ayant une forme de manivelle et prolongeant les moyens de fixation 50.

Les moyens de fixation 50 comportent une gouttière courbe 54 délimitée par une surface supérieure 56 bordée par deux parois latérales 58A, 58B. La courbure de la gouttière correspond à celle de la mâchoire dans la zone de fixation du dispositif de repérage.

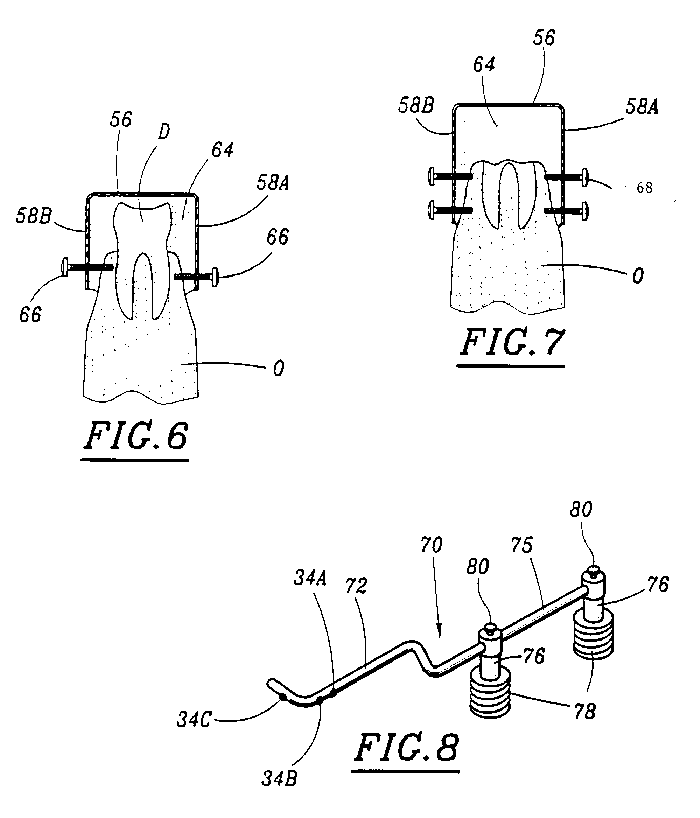

Les parois latérales 58A, 58B comportent, comme représenté sur la figure 5 par exemple, des trous taraudés 60 dans certains desquels sont vissés des organes filetés 62 dont l'extrémité libre, reçue dans la gouttière, est destinée à prendre appui sur la mâchoire.

Dans l'exemple représenté sur la figure 5, deux vis traversent chaque paroi latérale et enserrent une dent D. Les extrémités libres des vis prennent alors appui directement sur les parois latérales des dents.

Un matériau souple de garnissage 64, par exemple une pâte, est avantageusement disposé à l'intérieur de la gouttière 54 afin de remplir l'espace entre les parois délimitant cette gouttière et la mâchoire.

On conçoit que le serrage des vis contre la dent assure la retenue de la gouttière sur la mâchoire.

La tige 53 présente un premier bras fixé sur la surface supérieure 56 de la gouttière. L'autre bras portant les points de référence 34A à 34C est décalé dans un plan parallèle et a une longueur telle qu'il s'étend entre les lèvres du patient, de sorte que les points 34A à 34C sont disposés à l'extérieur de la bouche. La tige 53 peut être venue de matière avec la gouttière 54.

Dans le mode de réalisation de la figure 6, des vis 66 sont prévues seulement à l'extrémité inférieure des parois latérales 58A, 58B. Ces vis sont destinées à être vissées directement dans l'os O de la mâchoire, la paroi supérieure de la gouttière reposant alors sur les dents par l'intermédiaire du matériau de garnissage 64.

Sur la figure 7, est représentée l'implantation d'un dispositif de repérage sur une gencive édentée. Dans ce cas, des vis de fixation 68, dont quatre sont représentées sur la figure 7, ont leurs extrémités directement reçues à l'intérieur de l'os O.

Sur la figure 8, est représenté un autre type de dispositif de repérage. Celui-ci comporte, comme précédemment, une tige 70 en forme de manivelle comportant sur l'un de ses bras 72 les trois points de référence 34A, 34B, 34C formés par des diodes émettrices.

L'autre bras 75 de la tige est reçu dans deux plots de support 76 prolongés par des organes filetés 78 formant embase. Ces derniers sont destinés à être reçus dans des douilles préalablement implantées dans la mâchoire du patient. Les organes filetés 78 présentent extérieurement un filetage complémentaire à celui des douilles.

Les plots 76 sont formés par exemple par des tiges en titane ou en carbone comportant dans leur partie supérieure, un passage transversal dans lequel est introduit le bras 75 de la tige. Une vis axiale 80 assure le maintien en position de la tige par rapport aux plots de support 76.

Après mise en place du dispositif de repérage 34 et du dispositif de marquage 41, une première marque 40 est désignée par le chirurgien à l'aide de l'extrémité pointue d'une sonde (non représentée) en forme de stylet. Celle-ci comporte comme l'outil 20, quatre éléments de positionnement permettant le positionnement de la sonde par rapport à l'ensemble des points de référence 34A à 34C à l'aide du dispositif de positionnement 12.

Lorsque l'extrémité de la sonde est en contact avec la marque, le chirurgien indique au dispositif de positionnement 12, par exemple par l'enfoncement d'une pédale, que l'extrémité de la sonde se situe à l'emplacement d'une marque. Ainsi, l'unité 30 de traitement d'images détermine la position exacte de la marque par rapport à l'ensemble des points de référence 34A à 34C à partir notamment de la distance séparant l'extrémité de la sonde des éléments de positionnement qu'elle porte.

Après désignation de la première marque 40, le chirurgien identifie parmi les images numériques mémorisées l'image numérique contenant la marque désignée. L'unité centrale de traitement d'informations 14 mémorise alors la position de l'image numérique identifiée par rapport à l'ensemble des points de référence 34A à 34C. Cette position est déterminée à partir de la position de la marque 40 désignée contenue dans l'image identifiée.

La même succession d'opérations est effectuée pour chacune des marques 40. Le dispositif de marquage 41 est ensuite retiré.

Les images identifiées et ainsi positionnées seront par la suite utilisées afin de permettre la visualisation des opérations en cours sur les moyens d'affichage 16.

A partir des images numériques mémorisées, l'unité centrale de traitement d'informations 14 engendre une ou plusieurs images numériques résultant de la combinaison des différentes images mémorisées. Les images engendrées affichées sur les moyens d'affichage 16 permettent une visualisation de la zone 18A à traiter.

Pendant la phase péropératoire, le chirurgien travaille avec l'outil 20, sur le manche duquel sont disposés les quatre éléments de positionnement 32 permettant au dispositif de positionnement 12 de déterminer en continu la position de la tête de travail de l'outil par rapport à l'ensemble des points de référence 34A à 34C.

Ainsi, les informations relatives à la position de la tête de travail de l'outil sont transmises par l'unité de traitement d'images 30 à l'unité centrale de traitement d'informations 14. Cette dernière connaît alors les positions par rapport à l'ensemble des points de référence 34A à 34C d'une part de la tête de travail de l'outil et d'autre part des différentes images numériques. Elle engendre alors un représentant graphique de la tête de l'outil qu'elle incorpore dans une ou plusieurs images numériques de la zone à traiter. La ou les images montrant la position du représentant graphique de l'outil par rapport à la zone à traiter de l'organe sont affichées en continu sur les moyens d'affichage 16. En pratique, l'image visible sur les moyens d'affichage 16 est renouvelée à intervalle régulier, toutes les secondes par exemple.

Ainsi, le chirurgien peut contrôler visuellement la position relative de la tête d'outil 20 par rapport à la mâchoire sur laquelle il travaille.

Les marques 40 ne sont utilisées que temporairement pour positionner avec précision les images numériques utilisées par rapport à l'ensemble des points de référence 34A à 34C. Ces marques 40 sont retirées avant les opérations de traitement chirurgical.

On conçoit que le recours à de telles marques 40 qui sont disposées directement sur la zone à traiter garantit une précision élevée dans le positionnement de la tête d'outil par rapport aux images numériques représentatives de la zone traitée. En effet, les images numériques utilisées pour l'affichage sont positionnées par rapport à l'ensemble des points de référence 34A à 34C à l'aide de chacune des marques, ces marques étant repérées avec précision par rapport à l'ensemble des points de référence 34A à 34C lors de leur désignation à l'aide de la sonde. De plus, les mouvements de la tête de l'outil sont repérés par rapport à ce même ensemble de points de référence 34A à 34C.

Le dispositif de repérage 34 étant solidaire de la mâchoire, les images fournies par l'installation de contrôle visuel sont insensibles aux mouvements de la mâchoire au cours de l'opération chirurgicale.

En effet, les mouvements de la tête de travail de l'outil sont repérés en continu par le dispositif 12 dans un référentiel mobile lié au dispositif de repérage 34 par rapport auquel les images numériques ont été préalablement positionnées.

On comprend que l'utilisation d'une telle installation de contrôle visuel nécessite d'équiper la mâchoire à traiter des éléments d'un nécessaire de repérage comportant d'une part le dispositif de repérage 34 et d'autre part le dispositif de marquage 41.

Les dispositifs de repérage 34 et de marquage 41 décrits ici sont des organes amovibles qui permettent de garantir une précision élevée dans la visualisation de la zone à traiter pendant l'opération chirurgicale. En particulier, les moyens mis en oeuvre selon l'invention permettent de fixer temporairement ces dispositifs directement sur la mâchoire, tout en étant utilisables par l'installation de contrôle visuel.

En particulier, le dispositif amovible de marquage 41 comportant des moyens de positionnement ultérieur de celui-ci dans la même position que celle qu'il occupait lors de la prise des images numériques permet au patient de mener une vie normale entre le moment où sont prises les images et le moment où le chirurgien procède à l'intervention.

En variante non représentée, le dispositif 12 de positionnement comporte un émetteur/récepteur de type sonique. Dans ce cas, les éléments de positionnement 32 et les points de référence 34A à 34C sont des émetteurs soniques et les caméras 28A à 28C sont remplacées par un récepteur/émetteur approprié situé à proximité du patient.

En outre, les dispositifs de marquage 41 et de repérage 34 peuvent être solidaires et comporter une unique gouttière sur laquelle sont fixés à la fois l'ensemble de marque 40 et l'ensemble de points de référence 34A, 34B, 34C.

## Revendications

1. Dispositif de marquage (41), caractérisé en ce qu'il comporte un ensemble de marques (40) pouvant apparaître sur une image produite par un dispositif d'imagerie médicale (10), des moyens (42) de support amovible dudit ensemble de marques (40) sur une mâchoire (18) d'un être vivant dans une position déterminée, et des moyens (47) de positionnement ultérieur dudit ensemble de marques (40) dans ladite position déterminée après un retrait du dispositif.

2. Dispositif de marquage (41) selon la revendication 1, caractérisé en ce que lesdits moyens de positionnement ultérieur comportent une empreinte permanente (48) d'une partie de la mâchoire (18) prise lors de la première installation du dispositif, ladite empreinte (48) pouvant être remise en place sur ladite partie de la mâchoire lors de l'installation ultérieure du dispositif afin d'assurer le positionnement ultérieur de l'ensemble de marques (40) dans ladite position déterminée.

3. Dispositif de marquage selon la revendication 2, caractérisé en ce que lesdits moyens de support comportent une gouttière (42) sur laquelle sont disposées lesdites marques (40), et lesdits moyens de positionnement ultérieur comportent un matériau plastiquement déformable (47) disposé dans ladite gouttière (42) pour la prise de ladite empreinte (48).

4. Dispositif de marquage selon l'une quelconque des revendications précédentes, caractérisé en ce qu'il comporte des moyens (46) de fixation amovible du dispositif.

5. Dispositif de marquage selon la revendication 4, caractérisé en ce que lesdits moyens de fixation comportent des organes de serrage (46) destinés à enserrer ladite mâchoire.

6. Dispositif de marquage selon les revendications 3 et 5 prises ensemble, caractérisé en ce que lesdits organes de serrage comportent des vis (46) montées dans des trous taraudés (45) traversant des parois latérales (44A, 44B) de la gouttière (42), l'extrémité libre des vis étant destinée à coopérer avec ladite mâchoire.

7. Dispositif de marquage selon l'une quelconque des revendications 4 à 6, caractérisé en ce qu'il comporte en outre un ensemble de points de référence, comportant des éléments (34A, 34B, 34C) détectables par un dispositif (12) de positionnement d'un objet dans l'espace, lequel ensemble de points de référence est porté par des moyens (42) de support dudit ensemble de marques (40) sur la mâchoire.

8. Dispositif de repérage (34), caractérisé en ce qu'il comporte d'une part un ensemble de points de référence, comportant des éléments (34A, 34B, 34C) détectables par un dispositif (12) de positionnement d'un objet dans l'espace, et d'autre part des moyens (62 ; 66; 78) de fixation mécanique de cet ensemble de points de référence sur une mâchoire (18) d'un être vivant.

9. Dispositif de repérage selon la revendication 8, caractérisé en ce qu'il comporte un prolongement rigide (53 ; 70) reliant lesdits éléments (34A, 34B, 34C ) auxdits moyens de fixation, lequel prolongement rigide est destiné à traverser la bouche de l'être vivant afin de déporter lesdits éléments à l'extérieur du corps dudit être vivant.

10. Dispositif de repérage selon la revendication 8 ou 9, caractérisé en ce que lesdits moyens de fixation comportent d'une part, une gouttière (54) destinée à chevaucher un secteur de la mâchoire dudit être vivant, et d'autre part, des organes de serrage (62 ; 66; 70) destinés à enserrer ladite mâchoire pour retenir la gouttière sur celle-ci.

11. Dispositif de repérage selon la revendication 10, caractérisé en ce que lesdits organes de serrage comportent des vis (62 ; 66 ; 70) montées dans des trous taraudés (60) traversant des parois latérales (58A, 58B) de la gouttière, l'extrémité libre des vis étant destinée à coopérer avec ladite mâchoire.

12. Dispositif de repérage selon la revendication 8 ou 9, caractérisé en ce que lesdits moyens de fixation comportent au moins un support fileté (78) destiné à être vissé dans une douille implantée dans la mâchoire dudit être vivant.

13. Dispositif de repérage selon l'une quelconque des revendications 8 à 12, caractérisé en ce qu'il comporte un ensemble de marques (40) pouvant apparaître sur une image produite par un dispositif d'imagerie médicale (10), lesdits moyens de fixation mécanique comportant des moyens (42) de support dudit ensemble de marques (40) sur ladite mâchoire dans une position déterminée et en ce qu'il comporte en outre des moyens (47) de positionnement ultérieur dudit ensemble de marques (40) dans ladite position déterminée après un retrait du dispositif.
